# EUROPEAN PATENT SPECIFICATION

(11) **EP 4 570 289 B1**
(45) Date of publication and mention of the grant of the patent: **18.02.2026**
(21) Application number: 24727134.9
(22) Date of filing: 09.04.2024
(51) Int. Cl.: A61M 16/00, A61M 16/20

(54) **MANUAL RESUSCITATOR APPARATUS**
MANUELLE WIEDERBELEBUNGSVORRICHTUNG
APPAREIL DE RÉANIMATION MANUELLE

(30) Priority: 26.10.2023 CN 202311400283
(43) Date of publication of application: 18.06.2025
(73) Proprietor: Guangzhou Landswick Medical Technologies Ltd., Guangzhou, Guangdong 510000 (CN)
(72) Inventor: SUN, Caixin, Guangzhou, Guangdong 510000 (CN); HUANG, Yueqi, Guangzhou, Guangdong 510000 (CN); ZHAO, Yuheng, Guangzhou, Guangdong 510000 (CN); DONG, Hui, Guangzhou, Guangdong 510000 (CN)
(74) Representative: Santarelli
(86) International application number: PCT/CN2024/086841
(87) International publication number: WO 2025/086559

(56) References cited:
- WO-A1-2019/077493
- CN-A- 105 771 049
- CN-A- 109 758 656
- CN-A- 110 878 858
- CN-A- 117 205 419
- CN-U- 201 862 094
- CN-U- 208 726 471
- CN-U- 211 474 980
- FR-A1- 2 988 004
- GB-A- 894 094
- US-A- 4 694 825
- US-A1- 2009 205 660
- US-A1- 2018 021 533

## Description

### Technical Field

The present invention relates to the field of medical devices, more specifically, to an artificial resuscitator device for controllable ventilation volume.

### Background Art

An artificial resuscitator, also known as a simple breathing apparatus, is primarily used to provide forced ventilation to patients without breathing and assist breathing-impaired patients. Compared to mouth-to-mouth respiration, it offers higher oxygen concentration and is easier to operate. In critical conditions where there is no time for intubation, a pressurized mask can be used to deliver oxygen directly, providing patients with sufficient oxygen supply to improve oxygen-deficient states.

Artificial resuscitators are characterized by their simple structure, quick and convenient operation, and portability. They mainly consist of an elastic breathing bag, a respirator, a breathing valve, a gas storage bag, a mask or tracheal tube interface, and an oxygen interface. Oxygen enters a spherical bag and a gas storage bag, and by manually pressing the bag, oxygen is pushed through the front valve into a mask or tracheal tube closely attached to the patient's nose or mouth to achieve artificial ventilation.

However, manual operation of the artificial resuscitator requires the operator to manually press the bag for air supply, making it difficult to effectively control the ventilation volume. Even when operated by trained medical personnel, maintaining an appropriate ventilation volume for an extended period is challenging.

Therefore, there is a need to propose an artificial resuscitator device for controllable ventilation volume to at least partially address the issues in the existing technology.
FR2988004A1 discloses a device for manual resuscitator, including a self-inflating balloon, a flow rate regulation means incorporating an overpressure valve to limit the insufflation pressure, an exhalation valve, flow measuring means, an interface means for connecting the inflator to a person in distress and a timing means for signaling to a user the times conducive to insufflation. The flow rate regulating means comprises a drawer movable in a slide according to the flow rate and air passages whose section is modulated by the position of the drawer. The drawer comprises first elastic means for connecting with the slide and a first restriction capable of generating a pressure drop in the air flow, the pressure drop generating a pressure driving the drawer against the first elastic means.
WO2019/077493A1 discloses a flowmeter device for adjusting pulmonary manual ventilation, comprising at least one air/oxygen/gas passage chamber (15) comprising at least one preferably digital flowmeter (4) for measuring the air flow/oxygen/gas entering the chamber, said flowmeter comprising at least one pressure detector (7) of each insufflation and at least one volume detector (6) of each insufflation, said device further comprising at least one valve (9) for overpressure dispersion, at least one volume overflow shut-off valve (8), said valves (8, 9) being controlled by a microprocessor (10) suitable for measuring and limiting and controlling at least the volume of insufflation for a patient by means of said valves of said device (100) according at least to operator's settings.
CN211474980U discloses an air circuit switch valve, including a valve body, an air inlet pipe and an air outlet pipe arranged inside the valve body, a switching valve installation cavity is further provided inside the valve body, the air inlet pipe, the switch valve installation cavity and the air outlet pipe are connected in sequence, a valve flap membrane is provided inside the switch valve installation cavity and divides the switch valve installation cavity into a drainage cavity and an opening and closing cavity, the air inlet pipe, the opening and closing cavity and the air outlet pipe are connected in sequence, the air inlet pipe is also provided with a drainage pipe connected to the switch valve installation cavity, a control valve is provided inside the drainage pipe, the drainage pipe is connected to the drainage cavity, and the air outlet pipe is provided with a flow sensor.

### Summary of the Invention

To at least partially address the above problems, the present invention provides an artificial resuscitator device, as claimed in appended claim 1, comprising: a gas control valve and a flow sensor set on an exhaust pipe of a respirator; a connecting tube branching off the exhaust pipe, with a three-way valve on the connecting tube that selectively communicates with the control side of the gas control valve, and a pressure relief opening on one side of the three-way valve that selectively communicates with the control side of the gas control valve; a control module for obtaining the current ventilation volume of the respirator based on the gas flow detected by the flow sensor, and controlling whether the three-way valve switches the gas path based on the current ventilation volume to open or close the exhaust pipe through the gas control valve.

The basis for the control module to control whether the three-way valve switches the gas path is to compare the obtained current ventilation volume of the respirator with the set ventilation volume. If the current ventilation volume of the respirator reaches the set ventilation volume, the control module switches the three-way valve to the locked gas path, connecting the exhaust pipe to the connecting tube, causing the control side of the gas control valve to be pressurized and the exhaust pipe to be closed; if the current ventilation volume of the respirator does not reach the set ventilation volume, the control module switches the three-way valve to the pressure relief gas path, connecting the control side of the gas control valve to the pressure relief opening, with the control side of the gas control valve not under pressure, keeping the exhaust pipe open.

Preferably, the respirator includes a gas storage bag with an inlet one-way valve at the inlet end and an outlet one-way valve at the outlet end connected to the exhaust pipe.

Preferably, additionally, it includes a frequency dynamic indication module for prompting the operator to perform ventilation operations based on the set breathing frequency and breathing time interval, while controlling the three-way valve to switch to the pressure relief gas path. After ventilation, when the ventilation volume of the respirator reaches the set ventilation volume and has not reached the next ventilation time point, the control module switches the three-way valve to the locked gas path.

Preferably, the exhaust pipe includes a first pipe section connected to the outlet end of the gas storage bag and a second pipe section connected to the gas guiding section. The gas control valve is located at the junction of the first pipe section and the second pipe section, with the connecting tube branching off the first pipe section and the flow sensor located on the second pipe section.

Preferably, one end of the first pipe section communicating with the second pipe section is located inside the second pipe section, forming an annular connecting section, with the gas control valve located near the end of the annular connecting section in the second pipe section.

The gas control valve includes a pressure chamber connected to the second pipe section at one end and to the connecting tube at the other end, with the three-way valve located at the connection between the connecting tube and the pressure chamber, and the pressure relief opening selectively communicating with the pressure chamber through the three-way valve. A diaphragm assembly A is located at the connection between the second pipe section and the pressure chamber, selectively sealing the port of the first pipe section inside the second pipe section. The control side of the diaphragm assembly A, near the pressure chamber, experiences a greater gas pressure area than the ventilation side near the annular connecting section.

Preferably, the gas control valve uses a diaphragm valve, including a valve seat for connecting the first pipe section and the second pipe section, with a valve cavity inside the valve seat separated into two communicating cavities by a partition. A valve cover is located on one side of the valve seat, with a diaphragm assembly B inside the valve cover for selectively sealing the communicating cavity connected to the first pipe section. The valve cover is connected to the connecting tube via a pressure tube on the side away from the valve seat, with the three-way valve located at the connection between the connecting tube and the pressure tube, and the pressure relief opening selectively communicating with the pressure tube through the three-way valve. The control side of the diaphragm assembly B, near the pressure tube, is the control side, and the side near the communicating cavity is the ventilation side.

Preferably, diaphragm assembly A includes a diaphragm body, with both sides of the outer ring area connected to the second pipe section and the pressure chamber by the first fixing ring and the second fixing ring.

Preferably, the first fixing ring is uniformly arranged with multiple first grooves near one side of the diaphragm body, and the second fixing ring is uniformly arranged with multiple second grooves near one side of the diaphragm body. The first grooves contain the first pressure sensor in contact with the diaphragm body, and the second grooves contain the second pressure sensor in contact with the diaphragm body.

The first pressure sensor and the second pressure sensor are both connected to the control module to sense the force on thediaphragm body on the ventilation side and the control side to determine if the diaphragm body is functioning properly. In the present disclosure, a controllable ventilation volume method for an artificial resuscitator device is also described, wherein said method comprises:
Obtaining the set frequency and set ventilation volume based on the user's required breathing frequency and single ventilation volume;
Controlling the exhaust pipe of the respirator to be in the open state, obtaining the set breathing time interval based on the set frequency;
Real-time monitoring the gas flow at the exhaust pipe, obtaining the current ventilation volume of the respirator, and determining if the current ventilation volume meets the set ventilation volume;
If the current ventilation volume of the respirator does not meet the set ventilation volume, keeping the exhaust pipe of the respirator open; if the current ventilation volume of the respirator meets the set ventilation volume, closing the exhaust pipe of the respirator and monitoring the ventilation time interval in real-time;
If the ventilation time interval does not reach the set breathing time interval, keeping the exhaust pipe of the respirator closed; if the ventilation time interval reaches the set breathing time interval, opening the exhaust pipe of the respirator and prompting the operator for the next ventilation operation.

Compared to the prior art, the present invention has the following beneficial effects:
The artificial resuscitator device of the present invention uses a three-way valve and gas control valve to control the opening or closing of the exhaust pipe, providing a switch valve for controlling the exhaust pipe. Compared to high-flow, high-power consumption switch valves, the present invention uses low-power, small-aperture three-way valves. The control module can indirectly control the opening and closing of the gas control valve by controlling the three-way valve, reducing the power consumption of the artificial resuscitator device. It can effectively provide feedback on manual respiratory actions, cut off ventilation exceeding the required tidal volume, achieve quantitative ventilation, and retain the advantages of traditional artificial resuscitators while being compact and portable.

Other advantages, objectives, and features of the artificial resuscitator device of the present invention will be partially illustrated in the following description and will be understood by those skilled in the art through research and practice of the invention in this field. The present disclosure further describes a controllable ventilation volume method (not claimed) for an artificial resuscitator device.

### Brief Description of the Drawings

The drawings are used to provide a further understanding of the present invention and constitute a part of the specification. They are used in conjunction with the embodiments of the present invention to explain the invention and do not limit the invention. In the drawings:
Figure 1 is a schematic diagram of the control of the artificial resuscitator device of the present invention;
Figure 2 is a schematic diagram of the gas path principle of the artificial resuscitator device of the present invention;
Figure 3 is a schematic diagram of the first structure of the gas control valve when the three-way valve in the artificial resuscitator device of the present invention switches to the pressure relief gas path;
Figure 4 is a schematic diagram of the first structure of the gas control valve when the three-way valve in the artificial resuscitator device of the present invention switches to the locked gas path;
Figure 5 is a schematic diagram of the pressure on the ventilation side and control side of diaphragm assembly A in the artificial resuscitator device of the present invention when the three-way valve switches to the locked gas path;
Figure 6 is a schematic diagram of the second structure of the gas control valve in the artificial resuscitator device of the present invention;
Figure 7 is a schematic diagram of the structure of diaphragm assembly A in the artificial resuscitator device of the present invention;

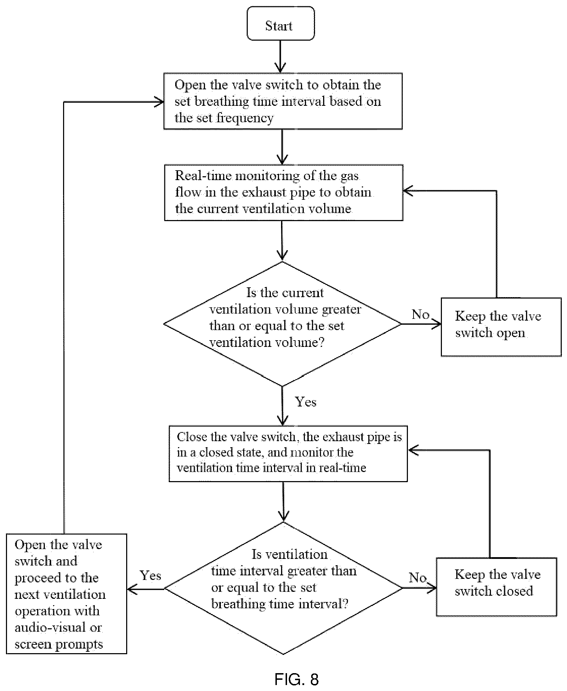

Figure 8 is a flowchart of the ventilation method with controllable ventilation volume of the artificial resuscitator device of the present invention.

### Detailed Description of Embodiments

Further detailed description of the present invention will be provided below in conjunction with the drawings and embodiments to enable those skilled in the art to implement the invention based on the description.

It should be understood that terms such as "comprising," "including," and "having" used in this document do not exclude the presence or addition of one or more other elements or combinations thereof.

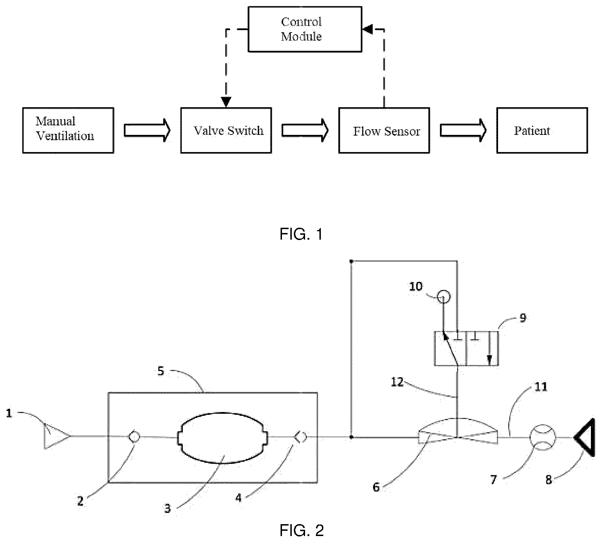

As shown in Figures 1 and 2, the present invention provides an artificial resuscitator device comprising: a gas control valve 6 and a flow sensor 7 set on the exhaust pipe 11 of the respirator 5; a connecting tube 12 is connected to the exhaust pipe 11, and a three-way valve 9 is provided on the connecting tube 12 to selectively communicate with the control side of the gas control valve 6, with a pressure relief opening 10 selectively communicating with the control side of the gas control valve 6 on one side of the three-way valve 9; a control module for obtaining the current ventilation volume of the respirator 5 based on the gas flow detected by the flow sensor 7 and controlling whether the three-way valve 9 switches the gas path based on the current ventilation volume to open or close the exhaust pipe 11 through the gas control valve 6.

Here, the ventilation volume refers to the single ventilation volume of the respirator 5, and the tidal volume refers to the amount of gas inhaled or exhaled during quiet breathing, which is related to age, gender, body surface area, breathing habits, and body metabolism; the set ventilation volume should match the patient's tidal volume;

When manually operating the respirator 5, the three-way valve 9 switches to the pressure relief gas path to relieve pressure on the control side of the gas control valve 6, thus opening the exhaust pipe 11. The flow sensor 7 can detect the gas flow each time the respirator 5 ventilates the patient, and the control module can receive real-time flow signals detected by the flow sensor 7 to obtain the current ventilation volume of the respirator 5. When the ventilation volume meets the patient's required tidal volume, the three-way valve 9 switches to the locked gas path to pressurize the control side of the control valve 6, closing the exhaust pipe to provide a quantified ventilation volume each time.

The gas control valve 6 and the three-way valve 9 form switch valves for controlling the opening or closing of the exhaust pipe 11. Traditional switch valves require high power consumption to ensure a large ventilation flow, which does not meet the portability requirements. In contrast, the present invention uses low-power, small-aperture three-way valves. By controlling the three-way valve 9, the control module can indirectly control the opening or closing of the gas control valve 6, reducing power consumption of the artificial resuscitator device. It can effectively provide feedback on manual respiratory actions, cut off ventilation volumes higher than the required tidal volume to achieve quantitative ventilation, and the device is compact and portable, retaining the advantages of traditional artificial resuscitators.

The control module controls whether the three-way valve 9 switches the gas path based on comparing the current ventilation volume of the respirator 5 with the set ventilation volume. If the current ventilation volume of the respirator 5 reaches the set ventilation volume, the three-way valve 9 switches to the locked gas path, connecting the exhaust pipe 11 to the connecting tube 12, pressurizing the control side of the gas control valve 6 to close the exhaust pipe 11. If the current ventilation volume of the respirator 5 does not reach the set ventilation volume, the three-way valve 9 maintains the current pressure relief gas path, connecting the connecting tube 12 to the pressure relief opening 10, and the control side of the gas control valve 6 is not pressurized, keeping the exhaust pipe 11 open.

The set ventilation volume is the tidal volume required by the patient. By monitoring the gas flow at the exhaust pipe 11 in real-time using the flow sensor 7, the current ventilation volume of the respirator 5 is obtained and compared with the set ventilation volume to control the switching of the three-way valve 9, ensuring that the single ventilation volume meets the patient's tidal volume requirements and preventing excessive ventilation that could harm the patient, achieving the function of quantitative ventilation.

The respirator 5 includes a gas storage bag 3, with an inlet one-way valve 2 at its intake end and an outlet one-way valve 4 at its outlet end connected to the exhaust pipe 11.

The gas storage bag 3 can be pressed by the operator to provide the required oxygen or air to the patient. The inlet one-way valve 2 allows gas to enter the gas storage bag 3 only from the intake end, while the outlet one-way valve 4 allows gas from the gas storage bag 3 to be discharged only to the exhaust pipe 11.

In one embodiment, it further includes a frequency dynamic indication module for prompting the operator to perform ventilation operations based on the set breathing time interval of the set frequency and controlling the three-way valve 9 to switch to the pressure relief gas path; after ventilation, i.e., when the ventilation volume of the respirator 5 reaches the set ventilation volume and has not reached the next ventilation time point, the control module controls the three-way valve 9 to switch to the locked gas path.

To provide a more accurate ventilation frequency for the patient, the ventilation frequency of the artificial resuscitator device is set based on the patient's respiratory frequency requirements, i.e., the set frequency. Specifically, based on the inhalation time interval of the set frequency, the operator is prompted to perform ventilation operations (pressing the gas storage bag 3). For example, if the respiratory frequency per minute is set to 20 breaths/min (set frequency) and each breath takes 3 seconds, the set breathing time interval is 3 seconds, with a prompt every 3 seconds for the operator to press the gas storage bag 3. The prompt can be displayed on the screen or through audio-visual alerts.

Furthermore, the frequency dynamic indication module is electrically connected to the control module. When the current ventilation volume of the respirator 5 reaches the set ventilation volume and has not reached the next ventilation time point (the ventilation time interval has not reached the preset ventilation time interval, no ventilation operation is prompted), the three-way valve 9 remains in the locked gas path to prevent accidental over-pressing of the gas storage bag 3 and excessive ventilation to the patient, ensuring safety.

In one embodiment, the exhaust pipe 11 includes a first pipe section 111 connected to the outlet end of the gas storage bag 3 and a second pipe section 112 connected to the gas guiding section 8; the gas control valve 6 is set at the connection between the first pipe section 111 and the second pipe section 112, the connecting tube 12 bypasses the first pipe section 111, and the flow sensor 7 is set on the second pipe section 112.

The gas guiding section 8 is a mask or tracheal tube used for patient ventilation.

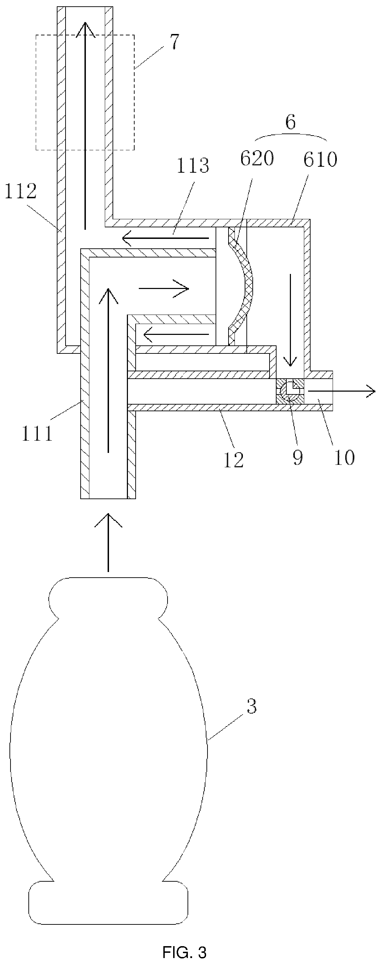

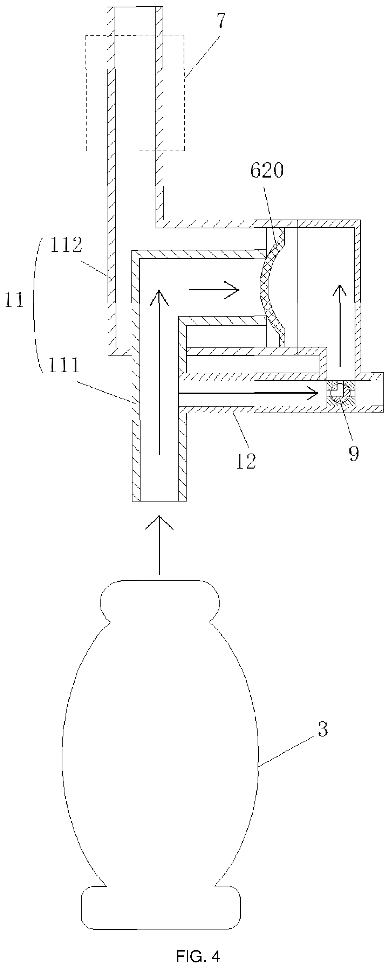

Furthermore, two structural forms of the gas control valve 6 are provided as follows:
As shown in Figures 3 and 4, the first structure of the gas control valve 6 includes one end of the first pipe section 111 connected to the inside of the second pipe section 112, forming an annular connecting section 113, and the gas control valve 6 is set near the end of the second pipe section 112 close to the annular connecting section 113;

The gas control valve 6 includes a pressure chamber 610 connected to the second pipe section 112 at one end and to the connecting tube 12 at the other end, the three-way valve 9 is set at the connection between the connecting tube 12 and the pressure chamber 610, and the pressure relief opening 10 selectively communicates with the pressure chamber 610 through the three-way valve 9; a diaphragm assembly A 620 is set at the connection between the second pipe section 112 and the pressure chamber 610, the diaphragm assembly A 620 can selectively seal the port of the first pipe section 111 inside the second pipe section 112; one side of the diaphragm assembly A 620 near the pressure chamber 610 is the control side, and the other side near the annular connecting section 113 is the ventilation side, with the control side of the diaphragm assembly A 620 having a larger area subjected to gas pressure than the ventilation side.

As shown in Figure 3, when ventilation is required for the patient, the control module controls the three-way valve 9 to switch to the pressure relief gas path, disconnecting the connecting tube 12 from the control side of the gas control valve 6, connecting the pressure relief opening 10 to the control side of the gas control valve 6, allowing the gas in the pressure chamber 610 to be released through the pressure relief opening 10, releasing the pressure. The gas discharged from the first pipe section 111 acts on the diaphragm of the diaphragm assembly A 620, causing the ventilation side of the diaphragm assembly A 620 to be subjected to greater force than the control side, deforming the diaphragm of the diaphragm assembly A 620 towards the control side, connecting the end of the first pipe section 111 to the annular connecting section 113, opening the switch valve, thus keeping the exhaust pipe 11 open;

As shown in Figure 4, when ventilation is not required for the patient, the control module controls the three-way valve 9 to switch to the locked gas path, connecting the connecting tube 12 to the control side of the gas control valve 6, connecting the first pipe section 111 to the pressure chamber 610, allowing the gas paths of the ventilation side and control side of the diaphragm assembly A 620 to be connected, with the pressure on both sides of the diaphragm assembly A 620 being consistent. Since the diaphragm assembly A 620 is designed with a larger pressure area on the control side than on the ventilation side, the control side experiences greater force than the ventilation side. At this point, the diaphragm of the diaphragm assembly A 620 deforms towards the ventilation side, sealing the end of the first pipe section 111, disconnecting the first pipe section 111 from the annular connecting section 113, closing the switch valve, and thus closing the exhaust pipe 11.

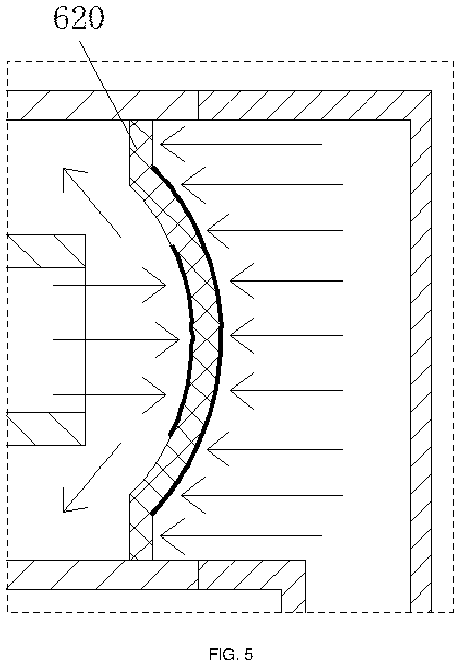

As shown in Figure 5, when the three-way valve 9 switches to the locked gas path, the schematic diagram shows the pressure on the ventilation side and control side of the diaphragm assembly A 620, with the thick black line on the right indicating the pressure on the control side and the thick black line on the left indicating the pressure on the ventilation side, where the pressure area on the ventilation side is smaller than that on the control side.

Through the above structure, the use of low-power, small-aperture three-way valves 9 in conjunction with the diaphragm assembly A 620 can achieve the opening or closing of the switch valve. The diaphragm assembly A 620 does not require electric control; by changing the gas path through the three-way valve 9, the forces on the ventilation side and control side of the diaphragm assembly A 620 can be altered to achieve the function of the switch valve. Compared to using high-power switch valves, the electric control power consumption is reduced, quantitative ventilation is achieved, and the portability of the artificial resuscitator is maintained.

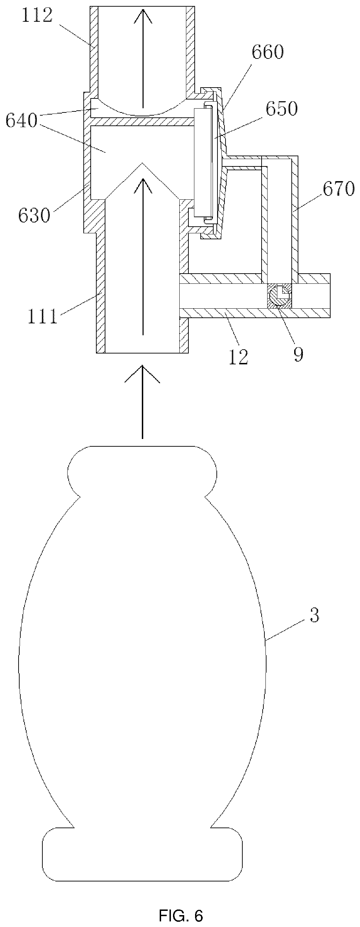

As shown in Figure 6, a second structure of the gas control valve 6 is illustrated. The gas control valve 6 adopts a diaphragm valve, comprising: a valve seat 630 for connecting the first pipe section 111 and the second pipe section 112, wherein the valve seat 630 is provided with a valve chamber, and a baffle inside the valve chamber divides it into two communicating cavities 640. Positioned on one side of the valve seat 630 is a valve cover 660, inside of which a diaphragm assembly B 650 is located. The diaphragm assembly B 650 selectively seals the communicating cavity 640 that communicates with the first pipe section 111. The valve cover 660, on the side away from the valve seat 630, communicates with the connecting tube 12 via a pressure tube 670, with the three-way valve 9 being positioned at the connection between the connecting tube 12 and the pressure tube 670. The pressure relief opening 10 selectively communicates with the pressure tube 670 through the three-way valve 9. The diaphragm assembly B 650 is the control side near the pressure tube 670 and the ventilation side near the communicating cavity 640.

The valve cover 660 seals one side of the valve chamber, with the diaphragm assembly B 650 sealingly connected to the valve cover 660. The diaphragm assembly B 650, away from the communicating cavity 640, forms the control side with the valve cover 660, while the side near the communicating cavity 640 serves as the ventilation side.

When ventilation to the patient is required, the control module switches the three-way valve 9 to the pressure relief air path, disconnecting the connecting tube 12 from the pressure tube 670. This action allows the pressure tube 670 to communicate with the pressure relief opening 10, enabling the control side to release pressure. Gas from the first pipe section 111 enters the communicating cavity 640 connected to it, exerting pressure on the ventilation side of the diaphragm assembly B 650, causing the diaphragm of the diaphragm assembly B 650 to deform towards the control side, thereby connecting the two communicating cavities 640 and opening the switching valve, with the exhaust pipe 11 in the open state.

When ventilation to the patient is not required, the control module switches the three-way valve 9 to the locked air path. The pressure tube 670 is connected to the pressure relief opening 10, and the connecting tube 12 is connected to the pressure tube 670. This connection allows the communicating cavity 640 connected to the first pipe section 111 to communicate with the pressure tube 670. The pressure on the ventilation side and the control side of the diaphragm assembly B 650 is equal, ensuring that the diaphragm assembly B 650 remains sealed.

To ensure the sealing of the diaphragm assembly B 650 to the communicating cavity 640, an elastic member that assists the diaphragm assembly B 650 in moving towards the ventilation side can be selectively placed on the control side. The combined force of the gas pressure and the elastic member on the control side is greater than the gas pressure on the ventilation side, thereby sealing the communicating cavity 640. The elastic force of the elastic member is small and does not cause the diaphragm of the diaphragm assembly B 650 to deform and move when acting alone.

Through the above structure, a low-power, small-aperture three-way valve 9 can be used in conjunction with the diaphragm assembly A 620 to achieve the opening or closing of the switching valve, enabling quantitative ventilation while retaining the portability of the manual resuscitator.

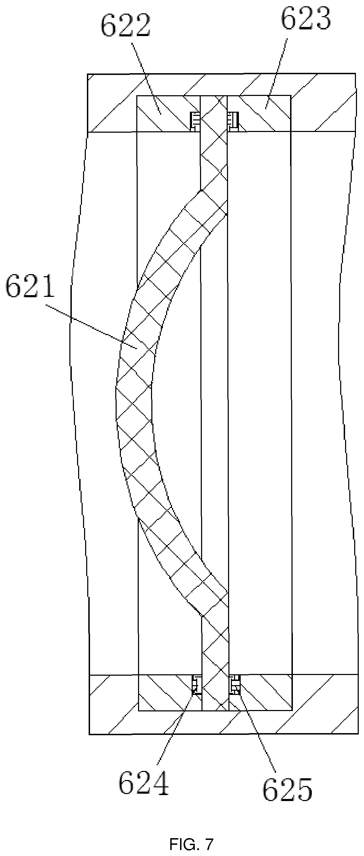

As shown in Figure 7, in one embodiment, the diaphragm assembly A 620 includes a diaphragm body 621, with its outer annular regions connected to the second pipe section 112 and the pressure chamber 610 by the first fixing ring 622 and the second fixing ring 623.

The outer annular region of the diaphragm body 621 is clamped between the first fixing ring 622 and the second fixing ring 623, which are sealingly connected to the second pipe section 112 or the pressure chamber 610, thereby securing the diaphragm body 621.

Additionally, the structure of the diaphragm assembly B 650 can be configured to include an installation shell that is sealably connected to the valve cover 660, with passages on both sides of the installation shell. The diaphragm body 621 is connected to the installation shell through the first fixing ring 622 and the second fixing ring 623 on its outer annular regions.

Furthermore, the first fixing ring 622 is uniformly provided with multiple first grooves on one side near the diaphragm body 621, and the second fixing ring 623 is uniformly provided with multiple second grooves on one side near the diaphragm body 621. The first grooves house the first pressure sensor 624 in contact with the diaphragm body 621, while the second grooves house the second pressure sensor 625 in contact with the diaphragm body 621.

The first pressure sensor 624 and the second pressure sensor 625 are both connected to the control module to sense the force on the ventilation side and the control side, determining the normal operation of the diaphragm body 621.

The first pressure sensor 624 is positioned on the ventilation side, and the second pressure sensor 625 is positioned on the control side. If the force on the ventilation side is greater than that on the control side, causing the diaphragm body 621 to move and deform towards the control side, the second pressure sensor 625 experiences compression, resulting in a change in the pressure value detected by the second pressure sensor 625. If the force on the control side is greater than that on the ventilation side, causing the diaphragm body 621 to move and deform towards the ventilation side, the first pressure sensor 624 experiences compression, leading to a change in the pressure value detected by the first pressure sensor 624.

During normal operation of the switching valve, when the diaphragm body 621 moves and deforms, the maximum pressure values detected by the first pressure sensor 624 and the second pressure sensor 625 (the maximum air pressure generated on the control side or the ventilation side when manually pressing the gas storage bag 3) should exceed the preset pressure value. The preset pressure value is the pressure required to deform the diaphragm body 621.

The movement and deformation of the diaphragm body 621 occur when switching the three-way valve 9 air paths. Therefore, each time the three-way valve 9 air paths are switched, the control module obtains the maximum pressure values detected by the first pressure sensor 624 and the second pressure sensor 625, compares them with the preset pressure value, and if the maximum pressure value is less than the preset pressure value, an alarm is issued. This indicates damage to the diaphragm body 621 or leakage on at least one side of the ventilation side and the control side, requiring cessation of use to prevent inappropriate ventilation and potential harm to the patient. In critical situations, the ventilation volume is displayed on the screen of the manual resuscitator device. If the diaphragm body 621 is working abnormally but still allows ventilation, the observed ventilation volume can be used to manually control the pressing and stopping of ventilation by the gas storage bag 3.

Prior to each use of this manual resuscitator device, a test can be conducted (without the gas guiding section 8 in contact with the patient) by manually pressing the gas storage bag 3 to check for any alarm prompts. If an alarm is triggered, it indicates that the manual resuscitator device is malfunctioning; if no alarm is triggered, the device can be used normally.

A method for controllable ventilation volume in a manual resuscitator device includes:
- Obtaining set frequency and set ventilation volume based on the user's required breathing frequency and single ventilation volume.
- Controlling the exhaust pipe 11 of the respirator 5 to be in the open state, obtaining the set breathing time interval based on the set frequency.
- Real-time monitoring the gas flow at the exhaust pipe 11 to determine the current ventilation volume of the respirator 5 and check if it meets the set ventilation volume.
- If the current ventilation volume does not meet the set ventilation volume, keeping the exhaust pipe 11 of the respirator 5 open; if the current ventilation volume meets the set ventilation volume, closing the exhaust pipe 11 of the respirator 5 and monitoring the ventilation time interval.
- If the ventilation time interval does not reach the set breathing time interval, keeping the exhaust pipe 11 of the respirator 5 closed; if the ventilation time interval reaches the set breathing time interval, opening the exhaust pipe 11 of the respirator 5 and prompting the operator to perform the next ventilation operation.

By utilizing the above method, frequency control is assisted, operators are reminded to perform ventilation operations, ensuring that the ventilation frequency to the patient aligns with their breathing frequency. The exhaust pipe 11 is controlled to open based on the set frequency time intervals, remaining closed at other unsuitable times to prevent improper ventilation by the respirator 5 and ensure safety during use.

Real-time monitoring of the gas flow at the exhaust pipe 11, integrating over time, allows for obtaining the single ventilation volume of the respirator 5. When the set ventilation volume is reached, ventilation is stopped by closing the exhaust pipe 11, achieving quantitative ventilation while preventing over-ventilation and ensuring the safety of patient ventilation.

In the description of the present invention, it should be understood that terms such as "center," "longitudinal," "lateral," "length," "width," "thickness," "top," "bottom," "front," "back," "left," "right," "vertical," "horizontal," "top," "bottom," "inner," "outer," "clockwise," "counterclockwise," "axial," "radial," "circumferential," and the like indicating orientation or positional relationships are based on the orientation or positional relationships shown in the drawings. These terms are used for ease of description and simplification, not to indicate or imply that the devices or components referred to must have specific orientations, be constructed or operated in specific orientations, and therefore should not be construed as limiting the present invention.

In the present invention, unless otherwise explicitly specified and limited, terms such as "mounting," "connecting," "linking," "fixing," and the like should be broadly interpreted. For example, it can be a fixed connection or a detachable connection, or integral; it can be a mechanical connection, an electrical connection, or communication with each other; it can be directly connected, or indirectly connected through an intermediate medium; it can be an internal connection of two components or an interaction relationship between two components, unless otherwise explicitly limited. For those skilled in the art, the specific meanings of the above terms in the present invention can be understood according to specific circumstances.

Although embodiments of the present invention are disclosed as above, it is not limited to the applications set forth in the specification and embodiments, it is fully applicable to various fields suitable for the present invention, and additional modifications can be readily accomplished by those familiar with the field, and therefore without departing from the scope of the invention defined in the appended claims. The present invention is not limited to the particular details with which the illustrations are shown and described herein and is defined in the appended claims.

## Claims

1. An artificial resuscitator device, comprising: a respirator (5) with an exhaust pipe (11), wherein the respirator (5) comprises a gas storage bag (3) with an inlet one-way valve (2) at the intake end and an outlet one-way valve (4) at the exhaust end, said exhaust end being connected to the exhaust pipe (11);
a gas control valve (6) having a control side;
a flow sensor (7) set on the exhaust pipe (11) of the respirator (5); a three way valve (9);
a connecting tube branching off the exhaust pipe (11), with the three-way valve (9) arranged on said connecting tube that selectively communicates with the control side of the gas control valve (6), and
a pressure relief opening (10) on one side of the three-way valve (9) that selectively communicates with the control side of the gas control valve (6); the artificial resuscitator device being **characterized in that** it further comprises
a control module for obtaining the current ventilation volume of the respirator (5) based on the gas flow detected by the flow sensor (7) and controlling whether the three-way valve (9) switches the gas path according to the current ventilation volume, to open or close the exhaust pipe (11); wherein
the exhaust pipe (11) includes a first pipe section (111) connected to the exhaust end of the gas storage bag (3) and a second pipe section (112) connected to a gas guiding section (8) for patient ventilation; wherein the gas control valve (6) is set at the junction of the first pipe section (111) and the second pipe section (112), with the connecting tube (12) branching off at the first pipe section (111), and the flow sensor (7) set on the second pipe section (112); and
wherein the control module is configured to control whether the three-way valve (9) switches the gas path based on the comparison of the current ventilation volume of the respirator (5) with the set ventilation volume; the control module being configured such that
if the current ventilation volume of the respirator (5) reaches the set ventilation volume, the control module switches the three-way valve (9) to the locked gas path, connecting the exhaust pipe (11) to the connecting tube (12), causing the gas control valve (6) to close the exhaust pipe (11); and
if the current ventilation volume of the respirator (5) does not reach the set ventilation volume, the control module switches the three-way valve (9) to the pressure relief gas path, connecting the control side of the gas control valve (6) to the pressure relief opening (10), and the control side of the gas control valve (6) remains unforced, keeping the exhaust pipe (11) open.

2. The artificial resuscitator device according to claim 1, further comprising: a frequency dynamic indication module for prompting the operator to perform ventilation operations based on a set breathing time interval frequency, the frequency dynamic indication module being configured to control the three-way valve (9) to switch to the pressure relief gas path through the control module, such that, after ventilation ends, when the ventilation volume of the respirator reaches the set ventilation volume and has not reached the next ventilation time node, the control module switches the three-way valve (9) to the locked gas path.

3. The artificial resuscitator device according to claim 1, wherein one end of the first pipe section (111) communicating with the second pipe section (112) is located inside the second pipe section (112), forming an annular connecting section (113), and the gas control valve (6) is set at the end of the second pipe section (112) near the annular connecting section (113); wherein the gas control valve (6) comprises a pressure chamber (610) connected to one end of the second pipe section (112) and to the connecting tube (12), with the three-way valve (9) being set at the connection between the connecting tube (12) and the pressure chamber (610), and the pressure relief opening (10) selectively communicating with the pressure chamber (610) through the three-way valve (9); a diaphragm assembly (A 620) being set at the connection between the second pipe section (112) and the pressure chamber (610), such that, in use, the diaphragm assembly (A 620) can selectively seal the port of the first pipe section (111) located inside the second pipe section (112) and the control side of diaphragm assembly (A 620) near the pressure chamber (610) is greater in area under gas pressure than the ventilation side near the annular connecting section (113).

4. The artificial resuscitator device according to claim 1, wherein the gas control valve (6) comprises a valve seat (630) for connecting the first pipe section (111) and the second pipe section (112), the valve seat comprising a valve cavity (630) separated into two communicating cavities (640) by a partition plate; a valve cover (660) set on one side of the valve seat (630), with a diaphragm assembly (B 650) inside the valve cover (660) for selectively sealing the communicating cavity (640) connected to the first pipe section (111); the valve cover (660) being configured to communicate with the connecting tube (12) through a pressure tube (670) on the side away from the valve seat, with the three-way valve (9) set at the connection between the connecting tube (12) and the pressure tube (670), and the pressure relief opening (10) selectively communicating with the pressure tube (670) through the three-way valve (9), such that the control side of diaphragm assembly (B 650) near the pressure tube (670) is greater in area under gas pressure than the ventilation side near the communicating cavity (640).

5. The artificial resuscitator device according to claim 3, wherein the diaphragm assembly (A 620) comprises a diaphragm main body (621) whose outer annular regions are connected to the second pipe section (112) and the pressure chamber (610) by a first fixing ring (622) and a second fixing ring (623).

6. The artificial resuscitator device according to claim 5, **characterized in that** the first fixing ring (622) is uniformly arranged with multiple first grooves near one side of the diaphragm body (621), and the second fixing ring (623) is uniformly arranged with multiple second grooves near one side of the diaphragm body (621); the first groove being equipped with a first pressure sensor (624) in contact with the diaphragm body (621), and the second groove being equipped with a second pressure sensor (625) in contact with the diaphragm body (621); the first pressure sensor (624) and the second pressure sensor (625) being both connected to a control module and configured to sense the force situation on the ventilation side and control side, in order to determine whether the diaphragm body (621) is functioning properly.

## Patentansprüche

1. Künstliche Beatmungsvorrichtung, umfassend: ein Beatmungsgerät (5) mit einem Abluftrohr (11), wobei das Beatmungsgerät (5) einen Gasspeicherbeutel (3) mit einem Einlass-Einwegventil (2) an dem Einlassende und einem Auslass-Einwegventil (4) an dem Auslassende umfasst, wobei das Auslassende mit dem Abluftrohr (11) verbunden ist; ein Gasregelventil (6) mit einer Regelseite;
einen Durchflusssensor (7), der an dem Abluftrohr (11) des Beatmungsgeräts (5) angebracht ist;
ein Dreiwegeventil (9);
ein Verbindungsrohr, das von dem Abluftrohr (11) abzweigt, wobei das Dreiwegeventil (9) an dem Verbindungsrohr angeordnet ist, das selektiv mit der Regelseite des Gasregelventils (6) in Verbindung steht, und
eine Druckentlastungsöffnung (10) auf einer Seite des Dreiwegeventils (9), die selektiv mit der Regelseite des Gasregelventils (6) in Verbindung steht; wobei die künstliche Beatmungsvorrichtung **dadurch gekennzeichnet ist, dass** sie ferner Folgendes umfasst:
ein Steuermodul zum Erhalten des aktuellen Beatmungsvolumens des Beatmungsgeräts (5) basierend auf dem von dem Durchflusssensor (7) detektierten Gasdurchfluss und zum Steuern, ob das Dreiwegeventil (9) den Gaspfad entsprechend dem aktuellen Beatmungsvolumen umschaltet, um das Abluftrohr (11) zu öffnen oder zu schließen; wobei
das Abluftrohr (11) einen ersten Rohrabschnitt (111), der mit dem Auslassende des Gasspeicherbeutels (3) verbunden ist, und einen zweiten Rohrabschnitt (112) enthält, der mit einem Gasführungsabschnitt (8) für die Patientenbeatmung verbunden ist; wobei das Gasregelventil (6) an der Verbindungsstelle des ersten Rohrabschnitts (111) und des zweiten Rohrabschnitts (112) angebracht ist, wobei das Verbindungsrohr (12) von dem ersten Rohrabschnitt (111) abzweigt, und der Durchflusssensor (7) an dem zweiten Rohrabschnitt (112) angebracht ist; und
wobei das Steuermodul so konfiguriert ist, dass es steuert, ob das Dreiwegeventil (9) den Gaspfad basierend auf dem Vergleich des aktuellen Beatmungsvolumens des Beatmungsgeräts (5) mit dem eingestellten Beatmungsvolumen umschaltet; wobei das Steuermodul so konfiguriert ist, dass,
wenn das aktuelle Beatmungsvolumen des Beatmungsgeräts (5) das eingestellte Beatmungsvolumen erreicht, das Steuermodul das Dreiwegeventil (9) auf den gesperrten Gaspfad umschaltet, wodurch das Abluftrohr (11) mit dem Verbindungsrohr (12) verbunden wird, wodurch veranlasst wird, dass das Gasregelventil (6) das Abluftrohr (11) verschließt; und
wenn das aktuelle Beatmungsvolumen des Beatmungsgeräts (5) das eingestellte Beatmungsvolumen nicht erreicht, das Steuermodul das Dreiwegeventil (9) auf den Druckentlastungsgaspfad umschaltet, wodurch die Regelseite des Gasregelventils (6) mit der Druckentlastungsöffnung (10) verbunden wird, und die Regelseite des Gasregelventils (6) unbelastet bleibt, wodurch das Abluftrohr (11) offen bleibt.

2. Künstliche Beatmungsvorrichtung nach Anspruch 1, ferner umfassend: ein Frequenz-Dynamik-Anzeigemodul zu der Aufforderung des Bedieners, Beatmungsvorgänge basierend auf einer eingestellten Atemzeitintervallfrequenz durchzuführen, wobei das Frequenz-Dynamik-Anzeigemodul so konfiguriert ist, dass es das Dreiwegeventil (9) steuert, um durch das Steuermodul auf den Druckentlastungsgaspfad umzuschalten, so dass, nachdem die Beatmung beendet ist, wenn das Beatmungsvolumen des Beatmungsgeräts das eingestellte Beatmungsvolumen erreicht und den nächsten Beatmungszeitknoten nicht erreicht hat, das Steuermodul das Dreiwegeventil (9) auf den gesperrten Gaspfad umschaltet.

3. Künstliche Beatmungsvorrichtung nach Anspruch 1, wobei sich ein Ende des ersten Rohrabschnitts (111), der mit dem zweiten Rohrabschnitt (112) in Verbindung steht, innerhalb des zweiten Rohrabschnitts (112) befindet, wodurch ein ringförmiger Verbindungsabschnitt (113) gebildet wird, und das Gasregelventil (6) an dem Ende des zweiten Rohrabschnitts (112) in der Nähe des ringförmigen Verbindungsabschnitts (113) angebracht ist; wobei
das Gasregelventil (6) eine Druckkammer (610) umfasst, die mit einem Ende des zweiten Rohrabschnitts (112) und mit dem Verbindungsrohr (12) verbunden ist, wobei das Dreiwegeventil (9) an der Verbindung zwischen dem Verbindungsrohr (12) und der Druckkammer (610) angebracht ist, und die Druckentlastungsöffnung (10) durch das Dreiwegeventil (9) selektiv mit der Druckkammer (610) in Verbindung steht; wobei eine Membranbaugruppe (A 620) an der Verbindung zwischen dem zweiten Rohrabschnitt (112) und der Druckkammer (610) so angebracht ist, dass die Membranbaugruppe (A 620) im Betrieb den Anschluss des ersten Rohrabschnitts (111), der sich innerhalb des zweiten Rohrabschnitts (112) befindet, selektiv abdichten kann und die Regelseite der Membranbaugruppe (A 620) in der Nähe der Druckkammer (610) in dem Bereich unter Gasdruck größer ist als die Beatmungsseite in der Nähe des ringförmigen Verbindungsabschnitts (113).

4. Künstliche Beatmungsvorrichtung nach Anspruch 1, wobei das Gasregelventil (6) einen Ventilsitz (630) zum Verbinden des ersten Rohrabschnitts (111) und des zweiten Rohrabschnitts (112), wobei der Ventilsitz einen Ventilhohlraum (630) umfasst, der durch eine Trennplatte in zwei in Verbindung stehende Hohlräume (640) getrennt ist; und einen Ventildeckel (660) umfasst, der auf einer Seite des Ventilsitzes (630) angebracht ist, wobei eine Membranbaugruppe (B 650) innerhalb des Ventildeckels (660) zum selektiven Abdichten des in Verbindung stehenden Hohlraums (640) mit dem ersten Rohrabschnitt (111) verbunden ist; wobei der Ventildeckel (660) so konfiguriert ist, dass er durch ein Druckrohr (670) auf der von dem Ventilsitz abgewandten Seite mit dem Verbindungsrohr (12) in Verbindung steht, wobei das Dreiwegeventil (9) an der Verbindung zwischen dem Verbindungsrohr (12) und dem Druckrohr (670) angebracht ist und die Druckentlastungsöffnung (10) durch das Dreiwegeventil (9) selektiv mit dem Druckrohr (670) in Verbindung steht, so dass die Regelseite der Membranbaugruppe (B 650) in der Nähe des Druckrohrs (670) in dem Bereich unter Gasdruck größer ist als die Beatmungsseite in der Nähe des in Verbindung stehenden Hohlraums (640).

5. Künstliche Beatmungsvorrichtung nach Anspruch 3, wobei die Membranbaugruppe (A 620) einen Membranhauptkörper (621) umfasst, dessen äußere ringförmige Bereiche durch einen ersten Befestigungsring (622) und einen zweiten Befestigungsring (623) mit dem zweiten Rohrabschnitt (112) und der Druckkammer (610) verbunden sind.

6. Künstliche Beatmungsvorrichtung nach Anspruch 5, **dadurch gekennzeichnet, dass** der erste Befestigungsring (622) einheitlich mit mehreren ersten Nuten in der Nähe einer Seite des Membrankörpers (621) angeordnet ist und der zweite Befestigungsring (623) einheitlich mit mehreren zweiten Nuten in der Nähe einer Seite des Membrankörpers (621) angeordnet ist; wobei die erste Nut mit einem ersten Drucksensor (624) in Kontakt mit dem Membrankörper (621) ausgestattet ist, und die zweite Nut mit einem zweiten Drucksensor (625) in Kontakt mit dem Membrankörper (621) ausgestattet ist;
wobei der erste Drucksensor (624) und der zweite Drucksensor (625) beide mit einem Steuermodul verbunden und so konfiguriert sind, dass sie die Lastsituation auf der Beatmungsseite und der Regelseite erfassen, um zu bestimmen, ob der Membrankörper (621) ordnungsgemäß funktioniert.

## Revendications

1. Dispositif de réanimation artificielle, comportant :
un respirateur (5) avec un tuyau d'échappement (11), dans lequel le respirateur (5) comporte un sac de stockage de gaz (3) avec une vanne unidirectionnelle d'entrée (2) à l'extrémité d'admission et une vanne unidirectionnelle de sortie (4) à l'extrémité d'échappement, ladite extrémité d'échappement étant raccordée au tuyau d'échappement (11) ;
une vanne de commande de gaz (6) ayant un côté commande ; un capteur de débit (7) posé sur le tuyau d'échappement (11) du respirateur (5) ;
une vanne à trois voies (9) ;
un tube de raccordement bifurquant du tuyau d'échappement (11), avec la vanne à trois voies (9) agencée sur ledit tube de raccordement qui communique sélectivement avec le côté commande de la vanne de commande de gaz (6), et
une ouverture de décompression (10) d'un côté de la vanne à trois voies (9) qui communique sélectivement avec le côté commande de la vanne de commande de gaz (6) ; le dispositif de réanimation artificielle étant **caractérisé en ce qu'**il comporte en outre
un module de commande pour obtenir le volume de ventilation actuel du respirateur (5) sur la base du débit de gaz détecté par le capteur de débit (7) et commander si la vanne à trois voies (9) commute le trajet de gaz en fonction du volume de ventilation actuel, pour ouvrir ou fermer le tuyau d'échappement (11) ; dans lequel
le tuyau d'échappement (11) inclut une première section de tuyau (111) raccordée à l'extrémité d'échappement du sac de stockage de gaz (3) et une seconde section de tuyau (112) raccordée à une section de guidage de gaz (8) pour la ventilation du patient ; dans lequel la vanne de commande de gaz (6) est posée au niveau de la jonction de la première section de tuyau (111) et de la seconde section de tuyau (112), le tube de raccordement (12) bifurquant au niveau de la première section de tuyau (111), et le capteur de débit (7) étant posé sur la seconde section de tuyau (112) ; et dans lequel le module de commande est configuré pour commander si la vanne à trois voies (9) commute le trajet de gaz sur la base de la comparaison du volume de ventilation actuel du respirateur (5) avec le volume de ventilation réglé ; le module de commande étant configuré de sorte que
si le volume de ventilation actuel du respirateur (5) atteint le volume de ventilation réglé, le module de commande commute la vanne à trois voies (9) sur le trajet de gaz verrouillé, raccordant le tuyau d'échappement (11) au tube de raccordement (12), amenant la vanne de commande de gaz (6) à fermer le tuyau d'échappement (11) ; et
si le volume de ventilation actuel du respirateur (5) n'atteint pas le volume de ventilation réglé, le module de commande commute la vanne à trois voies (9) sur le trajet du gaz de décompression, raccordant le côté commande de la vanne de commande de gaz (6) à l'ouverture de décompression (10), et le côté commande de la vanne de commande de gaz (6) reste non forcé, maintenant le tuyau d'échappement (11) ouvert.

2. Dispositif de réanimation artificielle selon la revendication 1, comportant en outre : un module d'indication dynamique de fréquence pour inviter l'opérateur à effectuer des opérations de ventilation sur la base d'une fréquence d'intervalle de temps de respiration réglée, le module d'indication dynamique de fréquence étant configuré pour commander la vanne à trois voies (9) pour passer au trajet de gaz de décompression par l'intermédiaire du module de commande, de sorte qu'après la fin de la ventilation, lorsque le volume de ventilation du respirateur atteint le volume de ventilation réglé et n'a pas atteint le nœud de temps de ventilation suivant, le module de commande commute la vanne à trois voies (9) sur le trajet de gaz verrouillé.

3. Dispositif de réanimation artificielle selon la revendication 1, dans lequel une extrémité de la première section de tuyau (111) communiquant avec la seconde section de tuyau (112) est située à l'intérieur de la seconde section de tuyau (112), formant une section de raccordement annulaire (113), et la vanne de commande de gaz (6) est posée à l'extrémité de la seconde section de tuyau (112) près de la section de raccordement annulaire (113) ; dans lequel
la vanne de commande de gaz (6) comporte une chambre de pression (610) raccordée à une extrémité de la seconde section de tuyau (112) et au tube de raccordement (12), la vanne à trois voies (9) étant posée au niveau du raccordement entre le tube de raccordement (12) et la chambre de pression (610), et l'ouverture de décompression (10) communiquant sélectivement avec la chambre de pression (610) par l'intermédiaire de la vanne à trois voies (9) ; un ensemble de membrane (A 620) étant posé au niveau du raccordement entre la seconde section de tuyau (112) et la chambre de pression (610), de sorte que, en utilisation, l'ensemble de membrane (A 620) puisse sceller sélectivement l'orifice de la première section de tuyau (111) situé à l'intérieur de la seconde section de tuyau (112) et que le côté commande de l'ensemble de membrane (A 620) proche de la chambre de pression (610) soit plus grand dans la zone sous pression de gaz que le côté ventilation proche de la section de raccordement annulaire (113).

4. Dispositif de réanimation artificielle selon la revendication 1, dans lequel la vanne de commande de gaz (6) comporte un siège de vanne (630) pour raccorder la première section de tuyau (111) et la seconde section de tuyau (112), le siège de vanne comportant une cavité de vanne (630) séparée en deux cavités communicantes (640) par une plaque de séparation ; un couvercle de vanne (660) posé d'un côté du siège de vanne (630), avec un ensemble de membrane (B 650) à l'intérieur du couvercle de vanne (660) pour sceller sélectivement la cavité de communication (640) raccordée à la première section de tuyau (111) ; le couvercle de vanne (660) étant configuré pour communiquer avec le tube de raccordement (12) par l'intermédiaire d'un tube de pression (670) sur le côté éloigné du siège de vanne, avec la vanne à trois voies (9) posée au niveau du raccordement entre le tube de raccordement (12) et le tube de pression (670), et l'ouverture de décompression (10) communiquant sélectivement avec le tube de pression (670) par l'intermédiaire de la vanne à trois voies (9), de sorte que le côté commande de l'ensemble de membrane (B 650) proche du tube de pression (670) est plus grand dans la zone sous pression de gaz que le côté ventilation près de la cavité communicante (640).

5. Dispositif de réanimation artificielle selon la revendication 3, dans lequel l'ensemble de membrane (A 620) comporte un corps principal de membrane (621) dont les régions annulaires extérieures sont raccordées à la seconde section de tuyau (112) et à la chambre de pression (610) par une première bague de fixation (622) et une seconde bague de fixation (623).

6. Dispositif de réanimation artificielle selon la revendication 5, **caractérisé en ce que** la première bague de fixation (622) est uniformément agencée avec plusieurs premières rainures près d'un côté du corps de membrane (621) et la seconde bague de fixation (623) est uniformément agencée avec plusieurs secondes rainures près d'un côté du corps de membrane (621) ; la première rainure étant équipée d'un premier capteur de pression (624) en contact avec le corps de membrane (621) et la seconde rainure étant équipée d'un second capteur de pression (625) en contact avec le corps de membrane (621) ;
le premier capteur de pression (624) et le second capteur de pression (625) étant tous deux raccordés à un module de commande et configurés pour détecter la situation de force du côté ventilation et du côté commande, afin de déterminer si le corps de membrane (621) fonctionne correctement.
